(19) Europäisches Patentamt
European Patent Office
Office européen des brevets

(11) Veröffentlichungsnummer: **0 040 325**
**B1**

(12) # EUROPÄISCHE PATENTSCHRIFT

(45) Veröffentlichungstag der Patentschrift: **05.03.86**

(51) Int. Cl.[4]: **A 61 K 31/70**

(21) Anmeldenummer: **81102836.4**

(22) Anmeldetag: **14.04.81**

(54) **Verwendung von Adenosinderivaten zur Herstellung eines Psychopharmakons.**

(30) Priorität: **21.05.80 DE 3019322**

(43) Veröffentlichungstag der Anmeldung:
**25.11.81 Patentblatt 81/47**

(45) Bekanntmachung des Hinweises auf die Patenterteilung:
**05.03.86 Patentblatt 86/10**

(84) Benannte Vertragsstaaten:
**AT BE CH DE FR GB IT LI NL SE**

(56) Entgegenhaltungen:
**FR-A-2 132 811**

(73) Patentinhaber: **Merck Patent Gesellschaft mit beschränkter Haftung**
**Frankfurter Strasse 250**
**D-6100 Darmstadt (DE)**

(72) Erfinder: **Irmscher, Klaus, Dr.**
**Reuterallee 10**
**D-6100 Darmstadt (DE)**
Erfinder: **Jonas, Rochus, Dr.**
**Telemannweg 24**
**D-6100 Darmstadt (DE)**
Erfinder: **Uhl, Jürgen, Dr.**
**Odenwaldstrasse 49**
**D-6104 Seeheim-1 (DE)**
Erfinder: **Schorscher, Ernst, Dr.**
**Im Fiedlersee 1**
**D-6100 Darmstadt-Arheilgen (DE)**
Erfinder: **Skolnick, Phil, Dr.**
**Lab. of Bioorgan. Chem.**
**NIAMDD National Institutes of Health Building 4**
**Room 212 Bethesda, Md. 20205 (US)**

**EP 0 040 325 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung eines Adenosinderivats der allgemeinen Formel I

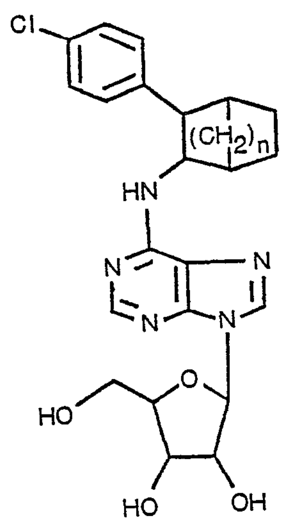

I

worin

n 1 oder 2 bedeutet oder eines seiner physiologisch unbedenklichen Säureadditionssalze zur Herstellung eines Psychopharmakons.

Die Bekämpfung von insbesondere psychischen Spannungszuständen mit Hilfe verschiedener Psychopharmaka ist bekannt. Vor allem werden Wirkstoffe aus der Gruppe der Benzodiazepine, z.B. Diazepam, für diese Zwecke verwendet. Trotz der weiten Verbreitung dieser Stoffe zeigen sie in ihrer Anwendung gewisse Nachteile. So wurden Appetitzunahme, Abnahme der Libido, Menstruationsstörungen, Schwindel und bei hohen Dosen Artikulationsstörungen beobachtet. Weiterhin wird die Alkoholtoleranz herabgesetzt.

Der vorliegenden Erfindung lag die Aufgabe zugrunde, andere Stoffe mit günstigeren Eigenschaften zur Verfügung zu stellen, die als Psychopharmaka bei der Behandlung von Spannungszuständen, z.B. als Anxiolytika und als Tranquillantia, verwendet werden können. Insbesondere sollten diese Stoffe eine von den Benzodiazepinen verschiedene Struktur besitzen, gut verträglich sein und die genannten Nebenwirkungen nicht oder nur in geringerem Ausmaß zeigen.

Diese Aufgabe wurde durch die Bereitstellung der Verbindungen der Formel I und ihrer pharmakologisch unbedenklichen Salze gelöst.

Es wurde gefunden, daß diese Substanzen bei guter Verträglichkeit wertvolle Wirkungen auf das zentrale Nervensystem besitzen. Vor allem zeigen sie zentral depressive Wirkungen. Im einzelnen treten insbesondere anxiolytische, antikonvulsive und muskelrelaxierende Eigenschaften auf.

Die anxiolytische Wirkung kann z.B. mit Hilfe eines unkonditionierten Konflikt-Tests nachgewiesen werden, wie er von A. S. Lippa et al. (Pharmacology, Biochemistry, Behavior, Band *9* (1978) Seiten 853—856) beschrieben wurde. So wurde z.B. nach dieser Methodik, jedoch bei einem Stromfluß von 700 µA, 30 Minuten nach intraperitonealer Verabfolgung von 20 mg/kg $N^6$-[2-(4-Chlorphenyl)-bicyclo[2,2,2]octyl-(3)]-adenosin (Ia; Formel I, n=2) an Ratten eine signifikante Wirkung beobachtet, verglichen mit Kontrolltieren, die nur Lösungsmittelgemisch injiziert bekommen hatten.

Die antikonvulsive Wirkung läßt sich z.B. gegenüber krampfauslösenden und tödlichen Dosen von Coffein oder Pentetrazol an Mäusen oder Ratten zeigen. Die muskelrelazierende Wirkung läßt sich z.B. nachweisen an Mäusen nach der Methodik von Irwin (Psychopharmakologia, Band 13 (1968) Seiten 222—257) oder an Ratten im Muskelrelaxations-Paartest [Methode vgl. H. Müller-Calgan et al., beschrieben in H. P. Zippel (Ed.), Memory and Transfer of Information, Plenum Press (New York—London), Seiten 97—100 (1973)] unter Verwendung eines Versuchsplans von A. Ribbentrop und W. Schaumann [Arzneimittelforschung, Band 15, Seiten 863—868 (1965)]. Weiter treten narkosepotenzierende Eigenschaften auf, die z.B. an Mäusen oder Ratten in Anlehnung an die Methode von Janssen et al. (Journal of Medicinal and Pharmaceutical Chemistry, Band 1, 1959, Seiten 281—297) nachgewiesen werden können, sowie auch narkoseverlängernde Wirkungen. Weiterhin wirken die Substanzen tranquillierend, was z.B. an der Spontanaktivität und dem Drohverhalten von Rhesusaffen [Methode vgl. H. Müller-Calgan, Activ. nerv. sup. (Praha), Band 16, Seiten 62—64 (1974)] beobachtet werden kann.

Außerdem kann eine sedierende Wirkung bei der Beobachtung der spontanen motorischen Aktivität von Mäusen festgestellt werden, beispielsweise mit Hilfe folgender Methodik: Gruppen von je 6 Mäusen werden in transparente Kunststoffkäfige gebracht und etwa 20 Minuten darin gelassen, um sich an die neue Umgebung zu adaptieren. Danach erhalten sie die Substanzen intraperitoneal. Die lokomotorische Aktivität der Tiere kann mit einem handelsüblichen Motilitäts-Meßgerät gemessen werden. Dabei löst die intraperitoneale Injektion von Ia eine dosis-abhängige Abnahme der spontanen motorischen Aktivität aus und erreicht innerhalb von 10 Min. ihr volles Ausmaß. Die Mäuse machen einen sedierten Eindruck, reagieren jedoch rasch auf Berührung. Nach Stimulation scheinen die Tiere rasch wieder in den sedierten Zustand zurückzukehren. Im Gegensatz dazu ziegen Tiere, die keine Injektion von Ia erhalten hatten, nach gleichartiger Stimulierung eine länger anhaltende Zunahme der lokomotorischen Aktivität. Die Wirkung von Ia auf die spontane Verhaltensaktivität ist langanhaltend.

Hinsichtlich der antikonvulsiven Wirkung kann eine synergistischer Effekt der Verbindungen der Formel I mit Benzodiazepinen, insbesondere Diazepam, beobachtet werden. So erzeugte eine intraperitoneale Gabe von subeffektiven Dosen Ia, gefolgt von subeffektiven Dosen Diazepam (d.h. Dosen, die jede für sich noch keinen antikonvulsiven Effekt zeigten) eine antikonvulsive Wirkung an Mäusen, deren Intensität von den verabfolgten Dosen Ia abhing.

Ferner wurde gefunden, daß eine Verabfolgung von Verbindungen der Formel I die Anzahl der Benzodiazepin-Rezeptoren im Gehirn von Mäusen vermehrt. Ein vergleichbarer Effekt durch Verabfolgung eines Wirkstoffes wurde bisher nicht beobachtet.

Dieser Effekt konnte z.B. wie folgt nachgewiesen werden: Mehrzweckmäusen im Gewicht von 20—25 g wurde durch intraperitoneale Injektion gelöstes Ia verabreicht. Die Lösung der Substanz in wässerigem Emulgatorgemisch wurde zu diesem Zweck 1:1 mit absolutem Ethanol vermischt, 1:10 mit phosphatgepufferter Kochsalzlösung verdünnt und die entsprechende Dosis in einem Volumen von höchstens 0,1 ml injiziert. Zu bestimmten Zeitpunkten wurden die Tiere dekapitiert, die "Vordergehirne" entnommen und in 0,32 molarer Rohrzuckerlösung homogenisiert.

Die Messung der Bindung von [$^3$H]-Diazepam (tritium-markiertem Diazepam) an die hypotonisch aus rohen Synaptosomen isolierten Zellmembranen erfolgte nach der Methode von S. M. Paul und P. Skolnick, (Science, Band *202* (1978), Seiten 892—894). Die intraperitoneale Injektion von 30 mg/kg Ia führte zu einem Anstieg der Bindung des tritiummarkierten Diazepams: Nach 10 Min. war ein leichter Anstieg zu beobachten, zwischen 30 und 240 Min. ein signifikanter Anstieg im Ausmaß von 110—125% der Kontrolle. Ein statistisch signifikanter Anstieg der Bindung des tritium-markierten Diazepams war bei Dosen zwischen 15 und 60 mg/kg zu beobachten, wenn 60 Min. nach Injektion gemessen wurde. Bei 120 mg/kg wurde eine geringfügige, aber statistisch signifikante Abnahme der [$^3$H]-Diazepam-Bindung beobachtet. Bei Anwendung eines Scatchard-Auswertungsverfahrens auf die Ergebnisse eines typischen Experiments (45 mg/kg, 30 Min. *vor* der Dekapitation verabreicht) ergab sich, daß die Zunahme der [$^3$H]-Diazepam-Bindung auf einen Anstieg der Benzodiazepin-Rezeptoren und nicht auf eine Änderung der scheinbaren Affinität zurückzuführen ist.

Die Verbindungen der Formel I und ihre pharmakologisch unbedenklichen Salze sind bekannt (vgl. z.B. DE—OS 21 17 577 und DE—OS 22 05 002). Auch einige ihrer pharmakologischen Wirkungen (Erhöhung des Coronardurchflusses, Vergrößerung des Sauerstoffpartialdrucks in coronarvenösem Blut, Wirksamkeit in einem intravasalen Schmerzmodell, derner kreislaufwirksame, lipolysehemmende und cholesterinspiegelsenkende Wirkungen) sind bereits bekannt.

Daß die Verbindungen außerdem und in erster Linie zentraldepressive Wirkungen besitzen, konnte den Angaben der Literatur jedoch nicht entnommen werden. Dieser Befund ist unerwartet und überraschend.

Die Verbindungen der Formel I können z.B. hergestellt werden durch Umsetzung von 6-Chlor-, 6-Brom- oder 6-Methyl-thio-9-(β-D-ribofuranosyl)-purin mit 2-Amino-3-p-chlorphenyl-bicyclo[2,2,2]octan oder 2-Amino-3-p-chlorphenyl-bicyclo[2,2,1]heptan oder durch Abspaltung von Schutzgruppen aus Verbindungen, die sonst der Formel I entsprechen, in denen jedoch OH-Gruppen in geschützter, z.B. veretherter oder veresterter Form vorliegen. Nähere Einzelheiten sind in der DE—OS 22 05 002 sowie in den nachstehenden Herstellungsbeispielen beschrieben.

Falls erwünscht, können die Verbindungen der Formel I durch Behandeln mit anorganischen oder organischen, vorzugsweise starken Säuren in ihre physiologisch unbedenklichen Säureadditionssalze übergeführt werden, z.B. in ihre Hydrochloride, Hydrobromide, Sulfate, Nitrate oder Methansulfonate.

Gegenstand der Erfindung ist somit die Verwendung eines Adenosinderivats der Formel I oder ihrer physiologisch unbedenklichen Salze zur Herstellung eines Psychopharmakons, insbesondere auf nicht-chemischem Wege. Hierbei kann es zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Die so erhaltenen Psychopharmaka können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den Verbindungen der Formel I nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate. Die Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine und/oder einen oder mehrere weitere psychopharmakologisch wirksame Stoffe, insebesondere Benzodiazepine.

Die Adenosinderivate der Formel I können bei der Bekämpfung von insbesondere psychischen Spannungszuständen (endogener oder exogener Ursache) des menschlichen oder tierischen Körpers, insbesondere von Erregung, Spannung, Angst, psychoneurotischen Störungen, vegetativer Dystonie, psychosomatischen Störungen

(insbesondere Herz, Kreislauf, Magen, Darm), Schlafstörungen, Muskelverspannungen (auch bei Rheuma), Krämpfen, Status epilepticus, ferner zur Erleichterung der Geburt und bei chirurgischen Interventionen verwendet werden.

Dabei werden die Adenosinderivate der Formel I in der Regel in Analogie zu bekannten, im Handel befindlichen Psychopharmaka, z.B. Diazepam, verabreicht, vorzugsweise in Dosierungen zwischen etwa 10 und 1000 mg, insbesondere zwischen 60 und 300 mg, vor allem zwischen 150 und 200 mg pro Dosierungseinheit. Die tägliche Dosierung liegt vorzugsweise zwischen etwa 0,2 und 20 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Herstellungsbeispiele:

Beispiel 1

Ein Gemisch aus 28,6 g 6-Chlor-9-(β-D-ribofuranosyl)-purin, 23,6 g 2-Amino-3-p-chlorphenyl-bicyclo[2,2,2]octan, 450 ml Dimethylformamid, 450 ml Isopropanol und 50 ml triethylamin wird 4 Tage bei 20° stehengelassen. Man dampft ein, löst in Chloroform, wäscht mit 1%iger wässeriger Essigsäure, dampft erneut ein, verreibt den Rückstand mit Ether und erhält $N^6$-[2-(4-Chlorphenyl)-bicyclo[2,2,2]octyl-(3)]-adenosin (Ia). F. 145° (Zersetzung). Rf 0,41 (Kieselgel; $CH_2Cl_2$ : $CH_3OH$ 9:1).

Beispiel 2

Analog Beispiel 1 erhält man aus äquimolekularen Mengen von 6-Brom-9-(β-D-ribofuranosyl)-purin und 2-Amino-3-p-chlorphenyl-bicyclo[2,2,1]-heptan das $N^6$-[2-(4-Chlorphenyl)-bicyclo[2,2,1]heptyl-(3)-adenosin (Ib). F. 145° (Zersetzung). Rf 0,43 (Kieselgel; $CH_2Cl_2$ : $CH_3OH$ 9:1).

Beispiel 3

Man kocht 72 g $N^6$-(2-p-Chlorphenyl-bicyclo[2,2,2]-octyl-3)-2',3',5'-tri-O-acetyl-adenosin (erhältlich aus 1-(Tri-O-acetyl-β-D-ribofuranosyl)-6-chlor-purin und 2-Amino-3-p-chlorphenyl-bicyclo[2,2,2]octan in Isopropanol) mit einer Lösung von 1 g Natrium in 1 l Methanol 90 Minuten lang. Dann wird mit Essigsäure neutralisiert, eingedampft und der Rückstand an 700 g Kieselgel chromatographiert (Chloroform:Methanol 99:1). Man erhält Ia.

Beispiel 4

a) Man kocht 1 g $N^6$-(2-p-Chlorphenyl-bicyclo[2,2,1]-heptyl-3)-adenin mit 10 ml Hexamethyldisilazan 15 Stunden, dampft anschließend ein und läßt den Rückstand mit 1,6 g 2,3,5-Tri-O-benzyl-D-ribofuranosylchlorid in 50 ml Acetonitril 3 Tage bei 20° stehen. Anschließend wird 2 Stunden gekocht. Das Lösungsmittel wird abgedampft und der Rückstand zwischen Wasser und Chloroform verteilt. Aus der Chloroformschicht erhält man 2',3',5'-Tri-O-benzyl-$N^6$-(2-p-chlorphenyl-bicyclo[2,2,1]heptyl-3)-adenosin als amorphen Sirup.

b) Das erhaltene Rohprodukt wird in 50 ml Ethanol aufgenommen und an 0,5 g Palladium-Kohle bei 20° unter Schütteln hydriert. Man filtriert den Katalysator ab, dampft ein und erhält Ib.

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1,5 kg Ia, 6 kg Lactose, 1,8 kg Kartoffelstärke, 0,3 kg Talk und 0,15 kg Magnesiumstearat wird in üblicher Weise zu Tabletten gepreßt, derart, daß jede Tablette 150 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 3 kg Ib in üblicher Weise in Hartgelatinekapseln, so daß jede Kapsel 300 mg Wirkstoff enthält.

Beispiel D: Ampullen

Eine Lösung von 1 kg Ia in 30 l zweifach destilliertem Wasser wird steril filtriert, in Ampullen abgefüllt, unter sterilen Bedingungen lyophilisiert und steril verschlossen. Jede Ampulle enthält 50 mg Wirkstoff.

**Patentansprüche**

1. Verwendung eines Adenosinderivats der allgemeinen Formel I

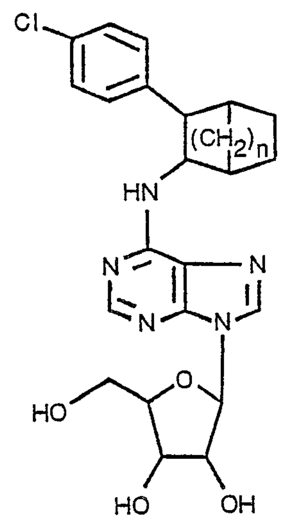

I

worin

n 1 oder 2 bedeutet oder eines seiner physiologisch unbedenklichen Säureadditionssalze zur Herstellung eines Psychopharmakons.

2. Verwendung eines Adenosinderivats der allgemeinen Formel I nach Anspruch 1 oder eines seiner physiologisch unbedenklichen Säureadditionssalze zusammen mit einem Benzodiazepinderivat zur Herstellung eines Psychopharmakons.

3. Verwendung eines Adenosinderivats der allgemeinen Formel I nach Anspruch 1 oder eines seiner physiologisch unbedenklichen Säureadditionssalze zusammen mit Diazepam zur Herstellung eines Psychopharmakons.

**Revendications**

1. Utilisation d'un dérivé de l'adénosine de formule générale I

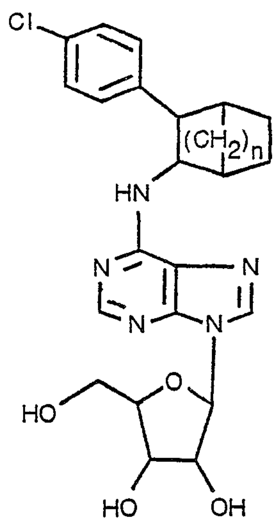

I

dans laquelle

n égal 1 ou 2, ou d'un de ses sels formés par addition avec des acides acceptables pour l'usage pharmaceutique pour la préparation d'un psychopharmacon.

2. Utilisation d'un dérivé de l'adénosine de formule générale I selon la revendication 1 ou de l'un de ses sels formés par addition avec des acides acceptables pour l'usage pharmaceutique, conjointement avec un dérive de benzodiazépine, pour la préparation d'un psychopharmacon.

3. Utilisation d'un dérivé de l'adénosine de formule générale I selon la revendication 1 ou de l'un de ses sels formés par addition avec des acides acceptables pour l'usage pharmaceutique, conjointement avec du Diazepam pour la préparation d'un psychopharmacon.

**Claims**

1. Utilization of an adenosine derivative of the general formula I

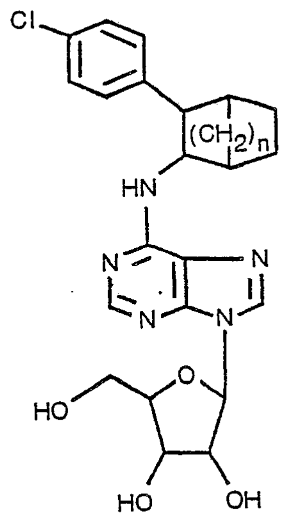

I

wherein

n is 1 or 2, or of one of its physiologically unobjectionable acid additions salts for the manufacture of a psychopharmacon.

2. Utilization of an adenosine derivative of the general formula I according to Claim 1 or of one of its physiologically unobjectionable acid addition salts together with a benzodiazepine derivative for the manufacture of a psychopharmacon.

3. Utilization of an adenosine derivative of the general formula I according to Claim 1 or of one of its physiologically unobjectionable acid addition salts together with diazepam for the manufacture of a psychopharmacon.